# EUROPÄISCHE PATENTSCHRIFT

(11) **EP 0 330 052 B1**
(45) Veröffentlichungstag und Bekanntmachung des Hinweises auf die Patenterteilung: **15.12.1993**
(21) Anmeldenummer: 89102505.8
(22) Anmeldetag: 14.02.1989
(51) Int. Cl.: A61K 31/50

(54) **Verwendung von Benzimidazolen zur Herstellung eines Arzneimittels mit antiischämischen Wirkungen am Herzen und dessen Kombinationen mit Beta-Blockern oder Bradycardica**
Use of benzimidazoles for preparing a medicine with an anti-ischemial activity on the heart, and combinations with betablockers or bradycardics
Utilisation de benzimidazoles pour la préparation de médicaments à activité anti-ischémique cardiaque et combinaisons avec des bêta-bloquants ou des agents bradycardisants

(30) Priorität: 24.02.1988 DE 3805635
(43) Veröffentlichungstag der Anmeldung: 30.08.1989
(73) Patentinhaber: Dr. Karl Thomae GmbH, D-88397 Biberach (DE)
(72) Erfinder: Dämmgen, Jürgen, Dr., D-7951 Sulmingen (DE); Seewaldt, Elke, Dr. Dipl.-Biol., D-7950 Biberach 1 (DE); Trach, Volker, Dr., D-7950 Biberach 1 (DE); Psiorz, Manfred, Dr. Dipl.-Chem., D-7957 Schemmerhofen-Alberweiler (DE); Reiffen, Manfred, Dr. Dipl.-Chem., D-7950 Biberach 1 (DE); Austel, Volkhard, Dr. Dipl.-Chem., D-7950 Biberach 1 (DE)

(56) Entgegenhaltungen:
- EP-A- 8 391
- EP-A- 0 156 163
- DE-A- 3 531 973
- DE-A- 3 535 949
- European journal of Pharmacology, 126, nos. 1-2, 1986, pages 21-30, Elsevier Science Publishers B.V. P.D. Verdouw et al.: 'Cardiovascular profile of pimobendan, a benzimidazole- pyridazinone derivative with vasodilating and inotropic properties'
- Naunyn-Schmiedberg's Arch. Pharmacol., Band 334, 1986, Seite R34, Zusammenfassung Nr. 138; CH. LINDER et al.: 'Influence of pimobendan on isovolumic UV relaxation in dogs with normal heart function and with ischaemic heart failure - comparison with milrinone'
- Journal of Cardiovascular Pharmacology, 11, no. 1, 1988, pages 100- 106, Raven Press, Ltd, New York, US H. Pouleur et al.: 'Effects of pimobendan (UD-CG 115) on the contractile function of the normal and 'Postischemic' canine myocardium'
- Eur. J. Clin. Invest., Band 17, 2 Teil 2, 1987, Seite A5, Zusammen- fassung Nr. 15; C. Hanet et al.: 'Effects of intropic stimulation with pimobendan (UD-CG 115) on postischemic left ventricular dysfunction'
- Am. Heart Assoc. Monogr., Band O, Nr. 124, 1986, Seite II-39, Zusammenfassung Nr. 154; O. GURNE et al.: 'Effects of pimobendan (UD-CG 115) on postischemic myocardial dysfunction'
- Eur. Heart J., Band 8, Ergänz. 2, 1987, Seite 73, Zusammenfassung Nr. 276 H.J. BRAND et al.: 'Hemodynamic effects of pimobendan given orally to patients with severe heart failure'

## Beschreibung

In der EP-B-0.008.391 werden u.a. in 2-Stellung substituierte 5-(5-Methyl-3-oxo-4,5-dihydro-2H-6-pyridazinyl)-benzimidazole der Formel 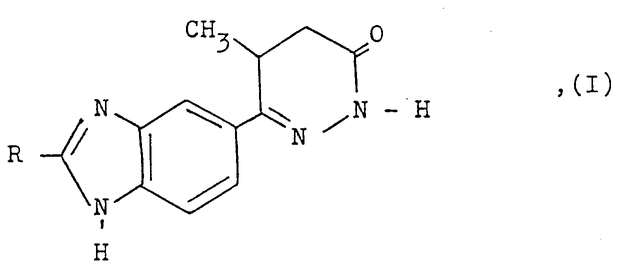
in der
R eine Alkylgruppe mit 1 bis 5 Kohlenstoffatomen, eine Hydroxyphenyl- oder Methoxyphenylgruppe bedeutet, deren 3H-Tautomere, deren optisch aktive Antipoden und deren physiologisch verträgliche Säureadditionssalze beschrieben, welche wertvolle pharmakologische Eigenschaften aufweisen, insbesondere neben einer antiviralen, Interferon-induzierenden und Ulcus-hemmenden Wirkung eine cardiovasculäre Wirkung, nämlich eine cardiotonische, blutdrucksenkende und/oder antithrombotische Wirkung.

Unter dem Begriff "cardiovasculäre Wirkungen" ist definitionsgemäß eine Wirkung betreffend das Herz und die Gefäße zu verstehen, die im vorliegenden Falle durch eine antithrombotische, cardiotonische und eine Wirkung auf den Blutdruck dokumentiert wird.

Aufgrund dieser pharmakologischen Eigenschaften eignen sich die Verbindungen der EP-B-0.008.391, deren 3H-Tautomere und deren optisch aktive Antipoden sowie deren physiologisch verträgliche Säureadditionssalze mit anorganischen oder organischen Säuren zur Behandlung der chronischen Herzinsuffizienz oder der Angina Pectoris und/oder zur Prophylaxe arterieller Thromboembolien und arteriellen Verschlußkrankheiten, sowie zur Behandlung von Ulcera und zur Bekämpfung von Viren und viralen Erkrankungen.

Deren Verwendbarkeit als Therapeutikum bei der chronischen Herzinsuffizienz beruht also auf deren cardiotonischer Wirkung sowie bei arteriellen Thromboembolien und Verschlußkrankheiten auf deren antithrombotischer Wirkung, insbesondere deren Thrombocytenwirkung. Folgerichtig wird daher in J. Cardiovasc. Pharmacol. 11, 100-106 (1988) für die Verbindung der allgemeinen Formel I, in der R die 4-Methoxyphenylgruppe darstellt (Pimobendan), eine günstige Wirkung auf die Funktion eines bereits ischämiegeschädigten und dadurch insuffizienten Herzmuskel beschrieben.

Es wurde nun gefunden, daß die vorstehend erwähnten Verbindungen unabhängig von den oben aufgeführten hämodynamischen und antithrombotischen Wirkungen eine antiischämische Wirkung am Herzen und damit eine kardioprotektive Wirkung aufweisen.

Gegenstand der vorliegenden Erfindung ist somit die neue antiischämische Wirkung am Herzen der vorstehend erwähnten Verbindungen der Formel I, die nicht auf einer hämodynamischen und antithrombitischen Wirkung beruht, insbesondere deren Verwendung bei der Prophylaxe und Behandlung des Herzinfarktes, insbesondere bei der Lyse des akuten Herzinfarktes, und die Herstellung hierfür geeigneter Arzneimittel.

Ein weiterer Gegenstand der vorliegenden Anmeldung ist ein Arzneimittel zur Prophylaxe des Herzinfarktes oder zur Behandlung des akuten Herzinfarktes, insbesondere bei der Lysetherapie des akuten Herzinfarktes, auf Basis einer Mischung bestehend aus einer Verbindung der vorstehend erwähnten Formel I und einem β-Blocker oder einem Bradycardicum neben Trägerstoffen und/oder anderen Zuschlagstoffen.

Bevorzugt sind hierbei diejenigen Verbindungen der Formel I, in der
R eine Methyl-, 2-Pentyl-, 4-Methoxyphenyl- oder 4-Hydroxyphenylgruppe darstellt,
insbesondere diejenigen Verbindungen der Formel I, in der
R eine 4-Methoxyphenyl- oder 4-Hydroxyphenylgruppe darstellt, deren 3H-Tautomere, deren optisch aktive Antipoden und deren physiologisch verträgliche Säureadditionssalze.

Beispielsweise kommen als β-Blocker Atenolol, Metoprolol, Pindolol, Penbutolol, Propanolol, Carazolol, Alprenolol, Bupranolol, Bisoprolol, Mepindolol, Metipranolol, Betaxolol, Acebutolol, Nadolol, Sotalol, Bunitrolol, Timolol und Oxprenolol
sowie als Bradycardicum Etafenon, Propafenon, Verapamil, Gallopamil, Anipamil, Diltiazem, eine in der EP-A-0.065.229 beschriebene Verbindung wie 1-(7,8-Dimethoxy-1,3,4,5-tetrahydro-2H-3-benzazepin-2-on-3-yl)-3-[N-methyl-N-(2-(3,4-dimethoxyphenyl)-ethyl)-amino]propan (Verbindung E),
eine in der EP-A-0.224.794 beschriebene Verbindung wie 3-[(N-(2-(3,4-Dimethoxyphenyl)-ethyl)-piperidin-3-yl)-methyl]-7,8-dimethoxy-1,3,4,5-tetrahydro-2H-3-benzazepin-2-on oder eine in der Deutschen Patentanmeldung P 37 17 561.0 vom 25. Juli 1987 beschriebenen Verbindung wie 3-[(N-(2-(Naphthyl-2)-ethyl)-piperidyl-3)-methyl]-7,8-dimethoxy-2-oxo-1,3,4,5-tetrahydro-2H-3-benzazepin,
3-[(N-(2-(5-Methyl-6-methoxy-naphthyl-2)-ethyl)-piperidyl-3)-methyl]-7,8-dimethoxy-2-oxo-1,3,4,5-tetrahydro-2H-3-benzazepin,
3-[2-(N-(2-(6-Methoxy-naphthyl-2)-ethyl)-piperidyl-2)-ethyl]-7,8-dimethoxy-2-oxo-1,3,4,5-tetrahydro-2H-3-benzazepin,
2-[(N-(2-(6-Methoxy-naphthyl-2)-ethyl)-hexahydro-azepinyl-3)-methyl]-6,7-methylendioxy-1-oxo-1,2,3,4-tetrahydro-isochinolin,
2-[(N-(2-(Naphthyl-2)-ethyl)-hexahydro-azepinyl-3)-methyl]-6,7-methylendioxy-1-oxo-1,2,3,4-tetrahydro-isochinolin,
2-[(N-(2-(3,4-Dimethoxy-phenyl)-ethyl)-piperidyl-3)-methyl]-6,7-dimethyl-1,2,3,4-tetrahydro-isochinolin,
2-[(N-(2-(3,4-Dimethoxy-phenyl)-ethyl)-piperidyl-3)-methyl]-6,7-dimethyl-1-oxo-1,2,3,4-tetrahydro-isochinolin,
2-[(N-(3-(4-Methoxy-phenoxy)-propyl)-piperidyl-3)-methyl]-6,7-dimethyl-1-oxo-1,2,3,4-tetrahydro-isochinolin,
3-[(N-(4-(Thienyl-2)-butyl)-piperidyl-3)-methyl]-7,8-dimethoxy-2-oxo-1,3,4,5-tetrahydro-2H-3-benzazepin,
3-[(N-(2-(Benzo[b]furyl-2)-ethyl)-piperidyl-3)-methyl]-7,8-dimethoxy-2-oxo-1,3,4,5-tetrahydro-2H-3-benzazepin und
3-[(N-(2-(Benzo[b]thienyl-3)-ethyl)-piperidyl-3)-methyl]-7,8-dimethoxy-2-oxo-1,3,4,5-tetrahydro-2H-3-benzazepin in Betracht.

Bevorzugte Arzneimittelkombinationen sind beispielsweise diejenigen, in denen beide Wirksubstanzen eine vergleichbare Halbwertzeit aufweisen, z.B. eine Kombination bestehend aus 2-(4-Methoxyphenyl)-5-(5-methyl-3-oxo-4,5-dihydro-2H-6-pyridazinyl)-benzimidazol (Verbindung A) und einem β-Blocker wie Atenolol, Betaxolol, Metoprolol, Timolol, Nadolol oder Propanolol oder
einem Bradycardicium wie Diltiazem,
1-(7,8-Dimethoxy-1,3,4,5-tetrahydro-2H-3-benzazepin-2-on-3-yl)-3-[N-methyl-N-(2-(3,4-dimethoxyphenyl)-ethyl)-amino]propan (Verbindung E) oder
3-[(N-(2-(3,4-Dimethoxyphenyl)-ethyl)-piperidin-3-yl)-methyl]-7,8-dimethoxy-1,3,4,5-tetrahydro-2H-3-benzazepin-2-on oder
aus 2-(4-Hydroxyphenyl)-5-(5-methyl-3-oxo-4,5-dihydro-2H-6-pyridazinyl)-benzimidazol (Verbindung B) und einem β-Blocker wie Metoprolol oder Pindolol oder
einem Bradycardicum wie Diltiazem,
1-(7,8-Dimethoxy-1,3,4,5-tetrahydro-2H-3-benzazepin-2-on-3-yl)-3-[N-methyl-N-(2-(3,4-dimethoxyphenyl)-ethyl)-amino]propan (Verbindung E) oder
3-[(N-(2-(3,4-Dimethoxyphenyl)-ethyl)-piperidin-3-yl)-methyl]-7,8-dimethoxy-1,3,4,5-tetrahydro-2H-3-benzazepin-2-on.

Verbindung A und deren Kombinationen eignen sich insbesondere für die perorale Applikation sowie Verbindung B und deren Kombinationen insbesondere für die intravenöse Applikation.

Die Einzeldosis am Erwachsenen liegt bei einer Verbindung der Formel I zwischen 0,1 und 10,0 mg, vorzugsweise jedoch zwischen 1,0 und 2,5 mg, 1- bis 2-mal täglich, um eine erfindungsgemäße Wirkung zu erzielen.

Demgegenüber kann die Einzeldosis des erfindungsgemäß eingesetzten β-Blockers oder Bradycardicums auf Grund ihrer verschiedenen Wirkungsstärken stark variieren. Diese beträgt beispielsweise pro Tag bei der Verwendung
von Atenolol 25 bis 100 mg, vorzugsweise jedoch 50 mg,
von Metoprolol 50 bis 150 mg, vorzugsweise 100 mg,
von Timolol 5 bis 15 mg, vorzugsweise 5 bis 10 mg,
von Nadolol 25 bis 80 mg, vorzugsweise 30 bis 60 mg,
von Propanolol 50 bis 100 mg, vorzugsweise 50 mg,
von Diltiazem 60 bis 300 mg, vorzugsweise jedoch 180 mg,
von 1-(7,8-Dimethoxy-1,3,4,5-tetrahydro-2H-3-benzazepin-2-on-3-yl)-3-[N-methyl-N-(2-(3,4-dimethoxyphenyl)-ethyl)-amino]propan (Verbindung E) 1 bis 20 mg, vorzugsweise 2 bis 10 mg, und von 3-[(N-(2-(3,4-Dimethoxyphenyl)-ethyl)-piperidin-3-yl)-methyl]-7,8-dimethoxy-1,3,4,5-tetrahydro-2H-3-benzazepin-2-on 0,1 bis 10 mg, vorzugsweise 0,5 bis 2,0 mg,
verteilt auf eine bzw. 2 Einzeldosen.

Die neu aufgefundene antiischämische Wirkung wurde beispielsweise an
- A =: 2-(4-Methoxyphenyl)-5-(5-methyl-3-oxo-4,5-dihydro-2H-6-pyridazinyl)-benzimidazol,
- B =: 2-(4-Hydroxyphenyl)-5-(5-methyl-3-oxo-4,5-dihydro-2H-6-pyridazinyl)-benzimidazol,
- C =: 2-Methyl-5-(5-methyl-3-oxo-4,5-dihydro-2H-6-pyridazinyl)-benzimidazol und
- D =: 3-[(N-(2-(3,4-Dimethoxyphenyl)-ethyl)-piperidin-3-yl)-methyl]-7,8-dimethoxy-1,3,4,5-tetrahydro-2H-3-benzazepin-2-on
wie folgt geprüft:

### 1. Beeinflussung der Infarktgröße an einem Ischämie/Reperfusionsmodell

Kaninchen (Russen beiderlei Geschlechts, Körpergewicht 2-2,5 kg) werden mit einem Bolus (40-60 mg/kg i.p.) und nachfolgender Dauerinfusion (10 |g/kg/h) Pentobarbital narkotisiert. Die Körpertemperatur wird bei 38°C gehalten. Nach endotrachealer Intubation werden die Tiere druckkonstant mit einer Mischung aus 50 % O₂ und 50 % N₂ beatmet. Die Kontrolle der Blutgase und des Blut pH erfolgt mit einem Blutgasanalyzer (ABL-2, Radiometer). Zur Messung des linksventrikulären Druckes wurde ein Druckwandler über die A-carotis in die linke Herzkammer geschoben. Der Aortendruck wird über einen via A. femoralis eingeführten Katheter erfaßt. Nach Eröffnung des Thorax im 5. linken Interkostalraum wird die linke absteigende Koronararterie für 60 Minuten ligiert und danach die Koronararterie wieder eröffnet. Nach 30minütiger Reperfusion wird die Arterie an derselben Stelle wieder verschlossen und das normal perfundierte Myokard mit Patentblau (in den linken Vorhof injiziert) angefärbt. Das Herz wird danach in drei Scheiben geschnitten und das bisher ungefärbte, vorher ischämische Gewebe, wird in einem Puffer mit Triphenyltetrazolium angefärbt. Überlebende Herzmuskelreale färben sich ziegelrot, während totes Gewebe bleich erscheint. Die Herzmuskelscheiben werden dann fotographiert und die Flächen mit Hilfe eines computergestützten Planimetriergerätes bestimmt.

Nach einer Äquilibrierungsperiode von 30 bis 60 Minuten wird die Prüfsubstanz intravenös injiziert und 10 Minuten danach die oben genannte Koronararterie verschlossen.
Antiischämisch wirksame Substanzen verkleinern den Anteil des ischämischen Myokards, der am Ende des Versuches infarziert ist.

Die nachfolgende Tabelle enthält die gefundenen Werte:

| Substanz | Dosis | % des ischämischen Myokards infarziert |
|---|---|---|
| A | 0,5 mg/kg | 34 |
| A + Atenolol | 0,5 + 0,3 mg/kg | 17 |
| A + D | 0,5 + 0,1 mg/kg | 16 |
| Kontrolle | - | 44 |
| Atenolol | 0,3 mg/kg | 63 |

### 2. Wirkung auf die kollateralabhängige Restperfusion des Myokards nach wiederholter vollständiger Okklusion des umschlingenden Astes der linken Koronararterie

Hunde (mischrassig, beiderlei Geschlechts, 20-30 kg Körpergewicht) werden mit Morphin (1 mg/kg s.c.) prämediziert und mit Pentobarbital (i.v. 20 mg/kg Bolus gefolgt von einer Dauerinfusion von 8 mg/kg) narkotisiert. Die Körpertemperatur wird mit einem Heizkissen bei 38°C gehalten. Nach endotrachealer Intubation werden die Tiere mit einem Gemisch von 25 % O₂ und 75 % N₂O beatmet.
Der linksventrikuläre Druck wird mit einem Mikrodruckwandler gemessen, während der Aortendruck über einen via A. femoralis vorgeschobenen Katheter erfaßt wird.
Nach Thoraxeröffnung im linken 5. Interkostalraum wird der Ramus circumflexus der linken Koronararterie freipärpariert und der Meßkopf eines elektromagnetischen Flowmeters darumgelegt. Zur Injektion von radioaktiven Mikrosphären (regionale Perfusionsmessung) wird der linke Vorhof kanüliert. Die Referenzprobe wird über die linke A. femoralis 2 Minuten lang bei einem Fluß von 6 ml/Minute gezogen. Die Registrierung der hämodynamischen Daten erfolgt auf einem IFD Polygraphen.

### Versuchsprotokoll:

Nach einer 30minütigen Äquilibrierungsphase wird der Ramus circumflexus 6 mal im Abstand von jeweils 30 Minuten für 4 Minuten verschlossen. Dabei wird während der 4. Minute eine regionale Perfusionsbestimmung durchgeführt. Die Perfusion des Versorgungsbereiches des Ramus circumflexus ist abhängig von koronaren Kollateralgefäßen. Für die Beurteilung der Kollateralperfusion werden die besonders ischämiegefährdeten subendocardialen Herzmuskelschichten herangezogen. Als Bezugswert vor Substanzgabe dient der jeweilige Flußwert der dritten Okklusion.

Die nachfolgende Tabelle enthält die gefundenen Werte:

| Substanz | Dosis i.v. \|g/kg | % Änderung des Kollateralflusses pro Herzschlag im subendocardialen ischämischen Myocard |
|---|---|---|
| A | 10 | + 85 |
| A | 30 | +100 |
| B | 95 | +182 |
| C | 10 | +101 |
| C | 30 | +178 |
| B + Atenolol | 95 + 300 | +192 |
| B + D | 95 + 100 | +262 |
| Atenolol | 300 | - 4 |
| D | 100 | + 67 |
| Kontrolle | - | - 5 |

Die erfindungsgemäß einsetzten Verbindungen sind gut verträglich. So weisen beispielsweise die Verbindungen A, B, D und E folgende aktute Toxizitäten auf:

| Substanz | LD₅₀ | |
|---|---|---|
| | i.v. mg/kg | p.o. mg/kg |
| A | 72 (Ratte) | 600 (Maus) |
| B | >100 (Ratte) | |
| D | 50 (Maus) | 720 (Maus) |
| E | 89 (Maus) | 1 350 (Maus) |

Die Toxizitäten der in den Kombinationen erfindungsgemäß verwendeten β-Blocker und Bradicardica sind literaturbekannt und in therapeutischen Dosen gut verträglich.

Wie bereits eingangs erwähnt, weisen die Verbindungen der Formel I, deren 3H-Tautomere, deren optisch aktive Antipoden und deren physiologisch verträgliche Säureadditionssalze cardioprotektive Wirkungen auf und eignen sich daher gegebenenfalls in Kombination mit einem β-Blocker oder einem Bradycardicum zur Verwendung bei der Prophylaxe und Behandlung des Herzinfraktes, insbesondere als Zusatztherapie der Lyse des aktuen Herzinfarktes, z.B. bei der Lyse mittels t-PA, rt-PA, Urokinase oder Streptokinase.

Zur pharmazeutischen Anwendung lassen sich hierzu die vorstehend genannten Wirkstoffe zusammen mit einem oder mehreren inerten üblichen Trägerstoffen und/oder Verdünnungsmitteln, z.B. mit Maisstärke, Milchzucker, Rohrzucker, mikrokristalliner Cellulose, Magnesiumstearat, Polyvinylpyrrolidon, Zitronensäure, Weinsäure, Wasser, Wasser/Ethanol, Wasser/Glyzerin, Wasser/Sorbit, Wasser/Polyethylenglykol, Propylenglykol, Cetylstearilalkohol, Carboxymethylcellulose oder fetthaltige Substanzen wie Hartfett, in übliche galenische Zubereitungen wie Tabletten, Dragées, Kapseln, Pulver, Suspensionen, Tropfen, Ampullen, Säfte oder Zäpfchen einarbeiten.

Die nachfolgende Beispiele sollen die Erfindung näher erläutern:

### Beispiel 1

### Kapseln mit 2 mg 2-(4-Methoxyphenyl)-5-(5-methyl-3-oxo-4,5-dihydro-2H-6-pyridazinyl)-benzimidazol

### Zusammensetzung:

| | |
|---|---|
| (01) Wirksubstanz | 2,00 mg |
| (02) Milchzucker | 95,00 mg |
| (03) Maisstärke | 40,00 mg |
| (04) Aerosil | 2,00 mg |
| (05) Magnesiumstearat | 1,00 mg |
| | 1̅4̅0̅,̅0̅0̅ m̅g̅ |

### Herstellung:

Die Stoffe (01) - (05) werden gemischt und in Kapseln der Kapselgröße 4 abgefüllt.

### Beispiel 2

### Tabletten mit 2,5 mg 2-(4-Methoxyphenyl)-5-(5-methyl-3-oxo-4,5-dihydro-2H-6-pyridazinyl)-benzimidazol mit Teilkerbe

### Zusammensetzung:

| | |
|---|---|
| (01) Wirksubstanz | 2,50 mg |
| (02) Milchzucker | 59,50 mg |
| (03) Maisstärke | 50,00 mg |
| (04) Polyvinylpyrrolidon | 5,00 mg |
| (05) Aerosil | 2,00 mg |
| (06) Magnesiumstearat | 1,00 mg |
| | 1̅2̅0̅,̅0̅0̅ m̅g̅ |

### Herstellung:

Die Stoffe (01) - (03) werden gemischt, mit einer Lösung von (04) in Ethanol granuliert, getrocknet, gesiebt, mit (05) + (06) gemischt und zu Tabletten mit 7 mm Durchmesser verpreßt.

### Beispiel 3

### Dragees mit 1 mg 2-(4-Methoxyphenyl)-5-(5-methyl-3-oxo-4,5-dihydro-2H-6-pyridazinyl)-benzimidazol

### Zusammensetzung:

| | |
|---|---|
| (01) Wirksubstanz | 1,00 mg |
| (02) Milchzucker | 61,00 mg |
| (03) Maisstärke | 50,00 mg |
| (04) Polyvinylpyrrolidon | 5,00 mg |
| (05) Aerosil | 2,00 mg |
| (06) Magnesiumstearat | 1,00 mg |
| | 1̅2̅0̅,̅0̅0̅ m̅g̅ |

### Herstellung:

Die Stoffe (01) - (03) werden gemischt, mit einer Lösung von (04) in Ethanol granuliert, getrocknet, gesiebt, mit (05) + (06) gemischt und zu Kernen von 7 mm Durchmesser verpreßt. Anschließend werden die Kerne nach üblichem Verfahren mit einer Hülle überzogen.

### Beispiel 4

### Tabletten mit 1,0 mg 2-(4-Methoxyphenyl)-5-(5-methyl-3-oxo-4,5-dihydro-2H-6-pyridazinyl)-benzimidazol und 50,0 mg Atenolol

### Zusammensetzung:

| | |
|---|---|
| (01) Wirksubstanz | 1,00 mg |
| (02) Atenolol | 50,00 mg |
| (03) Milchzucker | 47,00 mg |
| (04) Maisstärke | 70,00 mg |
| (05) Polyvinylpyrrolidon | 8,00 mg |
| (06) Aerosil | 3,00 mg |
| (07) Magnesiumstearat | 1,00 mg |
| | 1̅8̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 8 mm Durchmesser verpreßt.

### Beispiel 5

### Tabletten mit 0,5 mg 2-(4-Methoxyphenyl)-5-(5-methyl-3-oxo-4,5-dihydro-2H-6-pyridazinyl)-benzimidazol und 50,0 mg Atenolol

### Zusammensetzung:

| | |
|---|---|
| (01) Wirksubstanz | 0,50 mg |
| (02) Atenolol | 50,00 mg |
| (03) Milchzucker | 47,50 mg |
| (04) Maisstärke | 70,00 mg |
| (05) Polyvinylpyrrolidon | 8,00 mg |
| (06) Aerosil | 3,00 mg |
| (07) Magnesiumstearat | 1,00 mg |
| | 1̅8̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 8 mm Durchmesser verpreßt.

### Beispiel 6

### Tabletten mit 1,0 mg 2-(4-Methoxyphenyl)-5-(5-methyl-3-oxo-4,5-dihydro-2H-6-pyridazinyl)-benzimidazol und 30,0 mg Nadolol

### Zusammensetzung:

| | |
|---|---|
| (01) Wirksubstanz | 1,00 mg |
| (02) Nadolol | 30,00 mg |
| (03) Milchzucker | 67,00 mg |
| (04) Maisstärke | 70,00 mg |
| (05) Polyvinylpyrrolidon | 8,00 mg |
| (06) Aerosil | 3,00 mg |
| (07) Magnesiumstearat | 1,00 mg |
| | 1̅8̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 8 mm Durchmesser verpreßt.

### Beispiel 7

### Tabletten mit 1,0 mg 2-(4-Methoxyphenyl)-5-(5-methyl-3-oxo-4,5-dihydro-2H-6-pyridazinyl)-benzimidazol und 60,0 mg Nadolol

### Zusammensetzung:

| | |
|---|---|
| (01) Wirksubstanz | 1,00 mg |
| (02) Nadolol | 60,00 mg |
| (03) Milchzucker | 37,00 mg |
| (04) Maisstärke | 70,00 mg |
| (05) Polyvinylpyrrolidon | 8,00 mg |
| (06) Aerosil | 3,00 mg |
| (07) Magnesiumstearat | 1,00 mg |
| | 1̅8̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 8 mm Durchmesser verpreßt.

### Beispiel 8

### Kapseln mit 1,0 mg 2-(4-Methoxyphenyl)-5-(5-methyl-3-oxo-4,5-dihydro-2H-6-pyridazinyl)-benzimidazol und 50,0 mg Propanolol Retard

### Zusammensetzung:

In einer Schmelze (90° C) aus
19,00 mg Carnaubawachs und
110,00 mg Stearylalkohol werden
50,00 mg Propanolol dispergiert und
1,00 mg Magnesiumstearat zugemischt
1̅8̅0̅,̅0̅0̅ m̅g̅

### Herstellung:

Die Dispersion wird in einem geeigneten Gerät versprüht. Die Düse ist so zu wählen, daß Tröpfchen von 300 - 800 |m Durchmesser entstehen. Die Tröpfchen werden im freien Fall gegen einen auf ca. 5 - 8° C abgekühlten Luftstrom bewegt, so daß sie erstarren. Das so erhaltene Sprühgut wird mit 1 mg Magnesiumstearat gemischt und in Hartgelatine-Kapseln der Größe 3 abgefüllt.

### Beispiel 9

### Kapseln mit 1,0 mg 2-(4-Methoxyphenyl)-5-(5-methyl-3-oxo-4,5-dihydro-2H-6-pyridazinyl)-benzimidazol und 120,0 mg Diltiazem Retard

### Zusammensetzung:

In einer Schmelze (90° C) aus
29,00 mg Carnaubawachs und
180,00 mg Stearylalkohol werden
120,00 mg Diltiazem dispergiert und
1,00 mg Magnesiumstearat zugemischt
3̅3̅0̅,̅0̅0̅ m̅g̅

### Herstellung:

Die Dispersion wird in einem geeigneten Gerät versprüht. Die Düse ist so zu wählen, daß Tröpfchen von 300 - 800 |m Durchmesser entstehen. Die Tröpfchen werden im freien Fall gegen einen auf ca. 5 - 8° C abgekühlten Luftstrom bewegt, so daß sie erstarren. Das so erhaltene Sprühgut wird mit 1 mg Magnesiumstearat gemischt und in Hartgelatine-Kapseln der Größe 1 abgefüllt.

### Beispiel 10

### Kapseln mit 1,0 mg 2-(4-Methoxyphenyl)-5-(5-methyl-3-oxo-4,5-dihydro-2H-6-pyridazinyl)-benzimidazol und 100,0 mg Metoprolol Retard

### Zusammensetzung:

In einer Schmelze (90° C) aus
20,00 mg Carnaubawachs und
59,00 mg Stearylalkohol werden
100,00 mg Metoprolol dispergiert und
1,00 mg Magnesiumstearat zugemischt
1̅8̅0̅,̅0̅0̅ m̅g̅

### Herstellung:

Die Dispersion wird in einem geeigneten Gerät versprüht. Die Düse ist so zu wählen, daß Tröpfchen von 300 - 800 |m Durchmesser entstehen. Die Tröpfchen werden im freien Fall gegen einen auf ca. 5 - 8° C abgekühlten Luftstrom bewegt, so daß sie erstarren. Das so erhaltene Sprühgut wird mit 1 mg Magnesiumstearat gemischt und in Hartgelatine-Kapseln der Größe 3 abgefüllt.

### Beispiel 11

### Tabletten mit 1,0 mg 2-(4-Methoxyphenyl)-5-(5-methyl-3-oxo-4,5-dihydro-2H-6-pyridazinyl)-benzimidazol und 90,0 mg Diltiazem

### Zusammensetzung:

| | |
|---|---|
| (01) Wirksubstanz | 1,00 mg |
| (02) Diltiazem | 90,00 mg |
| (03) Milchzucker | 37,00 mg |
| (04) Maisstärke | 80,00 mg |
| (05) Polyvinylpyrrolidon | 8,00 mg |
| (06) Aerosil | 3,00 mg |
| (07) Magnesiumstearat | 1,00 mg |
| | 2̅2̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 9 mm Durchmesser verpreßt.

### Beispiel 12

### Tabletten mit 1,0 mg 2-(4-Methoxyphenyl)-5-(5-methyl-3-oxo-4,5-dihydro-2H-6-pyridazinyl)-benzimidazol und 10,0 mg Timolol

### Zusammensetzung:

| | |
|---|---|
| (01) Wirksubstanz | 1,00 mg |
| (02) Timolol | 10,00 mg |
| (03) Milchzucker | 51,00 mg |
| (04) Maisstärke | 50,00 mg |
| (05) Polyvinylpyrrolidon | 5,00 mg |
| (06) Aerosil | 2,00 mg |
| (07) Magnesiumstearat | 1,00 mg |
| | 1̅2̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 7 mm Durchmesser verpreßt.

### Beispiel 13

### Tabletten mit 1,0 mg 2-(4-Methoxyphenyl)-5-(5-methyl-3-oxo-4,5-dihydro-2H-6-pyridazinyl)-benzimidazol und 10,0 mg Betaxolol

### Zusammensetzung:

| | |
|---|---|
| (01) Wirksubstanz | 1,00 mg |
| (02) Betaxolol | 10,00 mg |
| (03) Milchzucker | 51,00 mg |
| (04) Maisstärke | 50,00 mg |
| (05) Polyvinylpyrrolidon | 5,00 mg |
| (06) Aerosil | 2,00 mg |
| (07) Magnesiumstearat | 1,00 mg |
| | 1̅2̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 7 mm Durchmesser verpreßt.

### Beispiel 14

### Tabletten mit 1,0 mg 2-(4-Methoxyphenyl)-5-(5-methyl-3-oxo-4,5-dihydro-2H-6-pyridazinyl)-benzimidazol und 100,0 mg Metoprolol

### Zusammensetzung:

| | |
|---|---|
| (01) Wirksubstanz | 1,00 mg |
| (02) Metoprolol | 100,00 mg |
| (03) Milchzucker | 27,00 mg |
| (04) Maisstärke | 80,00 mg |
| (05) Polyvinylpyrrolidon | 8,00 mg |
| (06) Aerosil | 3,00 mg |
| (07) Magnesiumstearat | 1,00 mg |
| | 2̅2̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 9 mm Durchmesser verpreßt.

### Beispiel 15

### Tabletten mit 1,0 mg 2-(4-Methoxyphenyl)-5-(5-methyl-3-oxo-4,5-dihydro-2H-6-pyridazinyl)-benzimidazol und 5,0 mg Timolol

### Zusammensetzung:

| | |
|---|---|
| (01) Wirksubstanz | 1,00 mg |
| (02) Timolol | 5,00 mg |
| (03) Milchzucker | 56,00 mg |
| (04) Maisstärke | 50,00 mg |
| (05) Polyvinylpyrrolidon | 5,00 mg |
| (06) Aerosil | 2,00 mg |
| (07) Magnesiumstearat | 1,00 mg |
| | 1̅2̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 7 mm Durchmesser verpreßt.

### Beispiel 16

### Tabletten mit 1,0 mg 2-(4-Methoxyphenyl)-5-(5-methyl-3-oxo-4,5-dihydro-2H-6-pyridazinyl)-benzimidazol (Wirksubstanz I) und 1,0 mg 1-(7,8-Dimethoxy-1,3,4,5-tetrahydro-2H-3-benzazepin-2-on-3-yl)-3-[N-methyl-N-(2-(3,4-dimethoxyphenyl)-ethyl)-amino]propan-hydrochlorid (Wirksubstanz II)

### Zusammensetzung:

| | |
|---|---|
| (01) Wirksubstanz I | 1,00 mg |
| (02) Wirksubstanz II | 1,00 mg |
| (03) Milchzucker | 60,00 mg |
| (04) Maisstärke | 50,00 mg |
| (05) Polyvinylpyrrolidon | 5,00 mg |
| (06) Aerosil | 2,00 mg |
| (07) Magnesiumstearat | 1,00 mg |
| | 1̅2̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 7 mm Durchmesser verpreßt.

### Beispiel 17

### Tabletten mit 1,0 mg 2-(4-Methoxyphenyl)-5-(5-methyl-3-oxo-4,5-dihydro-2H-6-pyridazinyl)-benzimidazol (Wirksubstanz I) und 2,5 mg 1-(7,8-Dimethoxy-1,3,4,5-tetrahydro-2H-3-benzazepin-2-on-3-yl)-3-[N-methyl-N-(2-(3,4-dimethoxyphenyl)-ethyl)-amino]propan-hydrochlorid (Wirksubstanz II)

### Zusammensetzung:

| | |
|---|---|
| (01) Wirksubstanz I | 1,00 mg |
| (02) Wirksubstanz II | 2,50 mg |
| (03) Milchzucker | 58,50 mg |
| (04) Maisstärke | 50,00 mg |
| (05) Polyvinylpyrrolidon | 5,00 mg |
| (06) Aerosil | 2,00 mg |
| (07) Magnesiumstearat | 1,00 mg |
| | 1̅2̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 7 mm Durchmesser verpreßt.

### Beispiel 18

### Tabletten mit 1,0 mg 2-(4-Methoxyphenyl)-5-(5-methyl-3-oxo-4,5-dihydro-2H-6-pyridazinyl)-benzimidazol (Wirksubstanz I) und 5,0 mg 1-(7,8-Dimethoxy-1,3,4,5-tetrahydro-2H-3-benzazepin-2-on-3-yl)-3-[N-methyl-N-(2-(3,4-dimethoxyphenyl)-ethyl)-amino]propan-hydrochlorid (Wirksubstanz II)

### Zusammensetzung:

| | |
|---|---|
| (01) Wirksubstanz I | 1,00 mg |
| (02) Wirksubstanz II | 5,00 mg |
| (03) Milchzucker | 56,00 mg |
| (04) Maisstärke | 50,00 mg |
| (05) Polyvinylpyrrolidon | 5,00 mg |
| (06) Aerosil | 2,00 mg |
| (07) Magnesiumstearat | 1,00 mg |
| | 1̅2̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 7 mm Durchmesser verpreßt.

### Beispiel 19

### Tabletten mit 1,0 mg 2-(4-Methoxyphenyl)-5-(5-methyl-3-oxo-4,5-dihydro-2H-6-pyridazinyl)-benzimidazol (Wirksubstanz I) und 0,5 mg 3-[(N-(2-(3,4-Dimethoxyphenyl)-ethyl)-piperidin-3-yl)-methyl]-7,8-dimethoxy-1,3,4,5-tetrahydro-2H-3-benzazepin-2-on-hydrochlorid (Wirksubstanz II)

### Zusammensetzung:

| | |
|---|---|
| (01) Wirksubstanz I | 1,00 mg |
| (02) Wirksubstanz II | 0,50 mg |
| (03) Milchzucker | 60,50 mg |
| (04) Maisstärke | 50,00 mg |
| (05) Polyvinylpyrrolidon | 5,00 mg |
| (06) Aerosil | 2,00 mg |
| (07) Magnesiumstearat | 1,00 mg |
| | 1̅2̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 7 mm Durchmesser verpreßt.

### Beispiel 20

### Tabletten mit 1,0 mg 2-(4-Methoxyphenyl)-5-(5-methyl-3-oxo-4,5-dihydro-2H-6-pyridazinyl)-benzimidazol (Wirksubstanz I) und 1,0 mg 3-[(N-(2-(3,4-Dimethoxyphenyl)-ethyl)-piperidin-3-yl)-methyl]-7,8-dimethoxy-1,3,4,5-tetrahydro-2H-3-benzazepin-2-on-hydrochlorid (Wirksubstanz II)

### Zusammensetzung:

| | |
|---|---|
| (01) Wirksubstanz I | 1,00 mg |
| (02) Wirksubstanz II | 1,00 mg |
| (03) Milchzucker | 60,00 mg |
| (04) Maisstärke | 50,00 mg |
| (05) Polyvinylpyrrolidon | 5,00 mg |
| (06) Aerosil | 2,00 mg |
| (07) Magnesiumstearat | 1,00 mg |
| | 1̅2̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 7 mm Durchmesser verpreßt.

### Beispiel 21

### Tabletten mit 1,0 mg 2-(4-Methoxyphenyl)-5-(5-methyl-3-oxo-4,5-dihydro-2H-6-pyridazinyl)-benzimidazol (Wirksubstanz I) und 2,0 mg 3-[(N-(2-(3,4-Dimethoxyphenyl)-ethyl)-piperidin-3-yl)-methyl]-7,8-dimethoxy-1,3,4,5-tetrahydro-2H-3-benzazepin-2-on-hydrochlorid (Wirksubstanz II)

### Zusammensetzung:

| | |
|---|---|
| (01) Wirksubstanz I | 1,00 mg |
| (02) Wirksubstanz II | 2,00 mg |
| (03) Milchzucker | 59,00 mg |
| (04) Maisstärke | 50,00 mg |
| (05) Polyvinylpyrrolidon | 5,00 mg |
| (06) Aerosil | 2,00 mg |
| (07) Magnesiumstearat | 1,00 mg |
| | 1̅2̅0̅,̅0̅0̅ m̅g̅ |

### Herstellung:

Die Stoffe (01) - (04) werden gemischt, mit einer Lösung von (05) in Ethanol granuliert, getrocknet, gesiebt, mit (06) + (07) gemischt und zu Tabletten mit 7 mm Durchmesser verpreßt.

### Beispiel 22

### Ampullen mit 1,0 mg 2-(4-Hydroxyphenyl)-5-(5-methyl-3-oxo-4,5-dihydro-2H-6-pyridazinyl)-benzimidazol-hydrochlorid und 5 mg Metoprolol

### Zusammensetzung:

| | |
|---|---|
| (01) Wirksubstanz | 1,00 mg |
| (02) Metoprolol | 5,00 mg |
| (03) Mannit | 100,00 mg |
| (04) 1N HCl ad pH 2,7 ca. | 2,4 µl |
| (05) Wasser f. Inj.zwecke ad | 2,0 ml |

### Herstellung:

(01) + (02) + (03) werden in Wasser gelöst, mit der Salzsäure wird auf pH 2,7 eingestellt, nach Sterilfiltration in 2 ml Ampullen abgefüllt. Sterilisieren 20 Minuten bei 120° C.

### Beispiel 23

### Ampullen mit 1,0 mg 2-(4-Hydroxyphenyl)-5-(5-methyl-3-oxo-4,5-dihydro-2H-6-pyridazinyl)-benzimidazol-hydrochlorid und 0,4 mg Pindolol

### Zusammensetzung:

| | |
|---|---|
| (01) Wirksubstanz | 1,00 mg |
| (02) Pindolol | 0,40 mg |
| (03) Mannit | 100,00 mg |
| (04) 1N HCl ad pH 2,7 ca. | 2,2 µl |
| (05) Wasser f. Inj.zwecke ad | 2,0 ml |

### Herstellung:

(01) + (02) + (03) werden in Wasser gelöst, mit der Salzsäure wird auf pH 2,7 eingestellt, nach Sterilfiltration in 2 ml Ampullen abgefüllt. Sterilisieren 20 Minuten bei 120° C.

### Beispiel 24

### Ampullen mit 1,0 mg 2-(4-Hydroxyphenyl)-5-(5-methyl-3-oxo-4,5-dihydro-2H-6-pyridazinyl)-benzimidazol-hydrochlorid (Wirksubstanz I) und 1,0 mg 1-(7,8-Dimethoxy-1,3,4,5-tetrahydro-2H-3-benzazepin-2-on-3-yl)-3-[N-methyl-N-(2-(3,4-dimethoxyphenyl)-ethyl)-amino]propan-hydrochlorid (Wirksubstanz II)

### Zusammensetzung:

| | |
|---|---|
| (01) Wirksubstanz I | 1,00 mg |
| (02) Wirksubstanz II | 1,00 mg |
| (03) Mannit | 100,00 mg |
| (04) 1N HCl ad pH 2,7 ca. | 2,3 µl |
| (05) Wasser f. Inj.zwecke ad | 2,0 ml |

### Herstellung:

(01) + (02) + (03) werden in Wasser gelöst, mit der Salzsäure wird auf pH 2,7 eingestellt, nach Sterilfiltration in 2 ml Ampullen abgefüllt. Sterilisieren 20 Minuten bei 120° C.

## Patentansprüche

1. Verwendung einer Verbindung der Formel 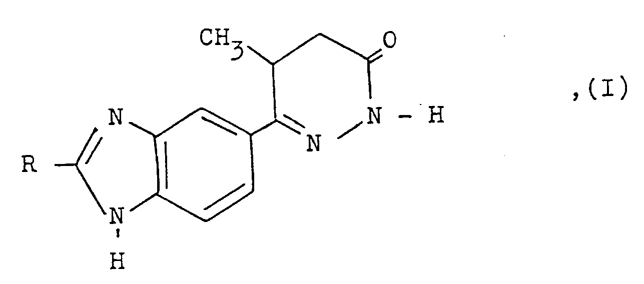 in der
R eine Alkylgruppe mit 1 bis 5 Kohlenstoffatomen, eine Hydroxyphenyl- oder Methoxyphenylgruppe bedeutet, deren 3H-Tautomere, deren optisch aktive Antipoden oder deren physiologisch verträgliche Säureadditionssalze zur Herstellung einer pharmazeutischen Zusammensetzung mit einer anti-ischämischen Wirkung am Herzen, die nicht auf einer hämodynamischen und antithrombotischen Wirkung beruht.

2. Verwendung einer Verbindung der Formel I gemäß Anspruch 1, in der
R eine Methyl-, 2-Pentyl-, 4-Methoxyphenyl- oder 4-Hydroxyphenylgruppe darstellt.

3. Verwendung einer Verbindung der Formel I gemäß Anspruch 1, in der
R eine 4-Methoxyphenyl- oder 4-Hydroxyphenylgruppe darstellt.

4. Verwendung von 2-(4-Methoxyphenyl)-5-(5-methyl-3-oxo-4,5-dihydro-2H-6-pyridazinyl)-benzimidazol gemäß Anspruch 1.

5. Verwendung von 2-(4-Hydroxyphenyl)-5-(5-methyl-3-oxo-4,5-dihydro-2H-6-pyridazinyl)-benzimidazol gemäß Anspruch 1.

6. Verwendung einer Verbindung der allgemeinen Formel I gemäß den Ansprüchen 1 bis 5 zur Herstellung eines Arzneimittels mit einer cardioprotektiven Wirkung, die nicht auf einer hämodynamischen und antithrombotischen Wirkung beruht.

7. Pharmazeutische Zusammensetzungen, enthaltend eine Verbindung der Formel I gemäß den Ansprüchen 1 bis 5 und einen β-Blocker oder ein Bradycardicum.

8. Verfahren zur Herstellung einer pharmazeutischen Zusammensetzung gemäß Anspruch 7, dadurch gekennzeichnet, daß eine Verbindung der Formel I gemäß den Ansprüchen 1 bis 5 und ein β-Blocker oder ein Bradycardicum in einen oder mehrere inerte Trägerstoffe und/oder Verdünnungsmittel eingearbeitet wird.

## Claims

1. Use of a compound of formula 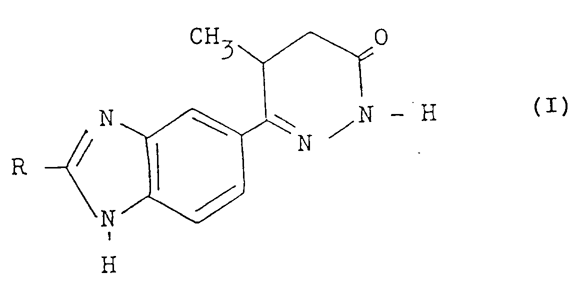 wherein
R represents an alkyl group with 1 to 5 carbon atoms, a hydroxyphenyl or methoxyphenyl group, the 3H tautomers, the optically active antipodes or the physiologically acceptable acid addition salts thereof, for preparing a pharmaceutical composition having an anti-ischaemic effect on the heart, which is not based on a haemodynamic and antithrombotic activity.

2. Use of a compound of formula I according to claim 1, wherein R represents a methyl, 2-pentyl, 4-methoxyphenyl or 4-hydroxyphenyl group.

3. Use a compound of formula I according to claim 1, wherein R represents a 4-methoxyphenyl or 4-hydroxyphenyl group.

4. Use of 2-(4-methoxyphenyl)-5-(5-methyl-3-oxo-4,5-dihydro-2H-6-pyridazinyl)-benzimidazole according to claim 1.

5. Use of 2-(4-hydroxyphenyl)-5-(5-methyl-3-oxo-4,5-dihydro-2H-6-pyridazinyl)-benzimidazole according to claim 1.

6. Use of a compound of general formula I according to claims 1 to 5 for preparing a pharmaceutical composition having a cardioprotective effect which is not based on a haemodynamic and antithrombotic effect.

7. Pharmaceutical compositions containing a compound of formula I according to claims 1 to 5 and a β-blocker or a bradycardiac agent.

8. Process for preparing a pharmaceutical composition according to claim 7, characterised in that a compound of formula I according to claims 1 to 5 and a β-blocker or a bradycardiac agent is incorporated in one or more inert carriers and/or diluents.

## Revendications

1. Utilisation d'un composé de formule 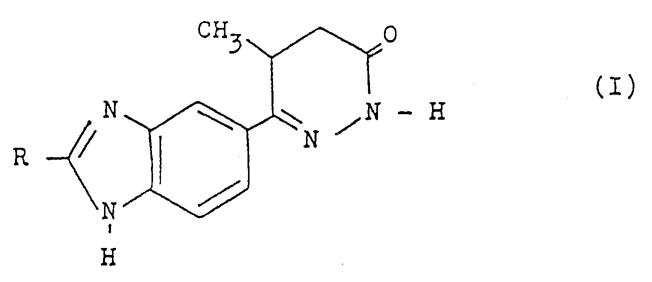 dans laquelle
R représente un groupe alkyle de 1 à 5 atomes de carbone, un groupe hydroxyphényle ou méthoxyphényle, de ses tautomères en 3H, de ses antipodes optiquement actifs ou de ses sels d'addition d'acide physiologiquement acceptables pour la préparation d'une composition pharmaceutique possédant une action anti-ischémique sur le coeur qui n'est pas basée sur une action hémodynamique et antithrombotique.

2. Utilisation d'un composé de formule I selon la revendication 1 dans laquelle R représente un groupe méthyle, 2-pentyle, 4-méthoxyphényle ou 4-hydroxyphényle.

3. Utilisation d'un composé de formule I selon la revendication 1 dans laquelle R représente un groupe 4-méthoxyphényle ou 4-hydroxyphényle.

4. Utilisation du 2-(4-méthoxyphényl)-5-(5-méthyl-3-oxo-4,5-dihydro-2H-6-pyridazinyl)-benzimidazole selon la revendication 1.

5. Utilisation du 2-(4-hydroxyphényl)-5-(5-méthyl-3-oxo-4,5-dihydro-2H-6-pyridazinyl)-benzimidazole selon la revendication 1.

6. Utilisation d'un composé de formule générale I selon les revendications 1 à 5 pour la préparation d'un médicament à action cardioprotectrice qui n'est pas basée sur une action hémodynamique et antithrombotique.

7. Compositions pharmaceutiques contenant un composé de formule I selon les revendications 1 à 5 et un β-bloquant ou un agent bradycardisant.

8. Procédé de préparation d'une composition pharmaceutique selon la revendication 7, caractérisé en ce qu'un composé de formule I selon les revendications 1 à 5 et un β-bloquant ou un agent bradycardisant sont incorporés dans un ou plusieurs véhicules et/ou diluants inertes.
